# EUROPEAN PATENT APPLICATION

(11) **EP 2 787 459 A1**
(43) Date of publication of application: **08.10.2014**
(21) Application number: 14162959.2
(22) Date of filing: 31.03.2014
(51) Int. Cl.: G06F 19/00

(54) **Method of monitoring nutritional intake by image processing**

(30) Priority: 05.04.2013 US 201313857427
(71) Applicant: Mutti, Christopher M., Hudson, MA 01749 (US)
(72) Inventor: Mutti, Christopher M., Hudson, MA 01749 (US)
(74) Representative: Hocking, Adrian Niall

(57) **Abstract**

A method of monitoring nutritional intake by image processing is completed through a fixed, and or, portable electronic computing device, where the computing device is communicably coupled with a database (2). At least one meal image is taken by the computing device, where the at least one meal image can be a three-dimensional image, a plurality of images, and a nutritional label image with at least one image (5). The at least one meal image enables the software to identify at least one component and a portion size of the at least one meal image (6). Then meal calories and nutritional facts are calculated and stored in order to monitor the intake food of an individual.

## Description

The current application claims a priority to the U.S. Non-Provisional Patent application serial number 13/857,427 filed on April 5, 2013.

### FIELD OF THE INVENTION

The present invention relates generally to a method to monitor nutritional intake. More particularly, a computerized method that incorporates advanced imagining features to capture an image of a meal and reference the image through an extensive database to determine the nutritional value, ingredients, portion, weight and automatically log the data to a user's account with the optional ability to produce three-dimensional scale model of the meal or any individual component thereof, which is used to assist in obtaining exact portion control.

### BACKGROUND OF THE INVENTION

There is a plurality of detrimental conditions that is linked with being overweight or obese. Overweight or obese individuals are more likely to suffer from hypertension, Dyslipidemia, and Type 2 diabetes, all of which are considered risk factors for cardiovascular disease. In the United States alone, 600,000 individuals die annually from cardiovascular disease and related illnesses while adding billions of dollars annually to cost of the nation's health care system. Half of the individuals who died annually were considered overweight or obese. With such alarming figures, concerned individuals have taken it upon themselves to find and implement effective methods to help them manage their weight and weight loss.

A well known and scientifically proven method for achieving weight loss and weight management goals is through active monitoring and recording of dietary habits. In one study, individuals that actively recorded their daily dietary habits were found to have lost twice as much weight as individuals that relied solely on diet and exercise. By actively recording ones dietary habits, gives individuals a better picture of their food intake and are able to adjust to meet their weight loss and management goals. Additionally, through the incorporation of nutrition facts, ingredient information panels, and bar codes found on all pre-packaged foods, individuals are able to keep track of their nutrient, ingredient, and portion intake such as calories, calories from fat, fat, saturated fat, trans fat, cholesterol, sodium, carbohydrates, dietary fiber, sugars, protein, vitamin A, vitamin C, calcium, iron, allergenic ingredients, genetically modified ingredients, portion weights or size, and as well as the percent daily values (DV) of the nutrient intake that is based on a given caloric diet.

Although it is common for many modern dietary programs to incorporate methods for tracking an individual's daily dietary intake, the vast majority rely on individuals to manually enter portion size, ingredients, and nutritional information. This is perhaps due to limitations in their methods or food databases from being unable to discern and quantify the nutritional value, portion size, and ingredients of a meal without a cumbersome, inconvenient, and impractical user input. This situation is most apparent when an individual is consuming prepared meals that lack the nutrition facts information panel and bar code. With recent advances in technology, specifically in three-dimensional (3D) imaging, (3D) Laser scanning, Point Cloud generation, Data Transmission via Internet Cloud, image recognition, logical form recognition, optical character recognition (OCR) ,three-dimensional (3D) print modeling, and mobile technology, the ability for software to recognize and log information, for a captured image, as well printing out the 3D scale models of the portion size for use in institution meal preparation such as, nursing homes, hospitals, professional & collegiate sport teams, motor sports teams, household, to aid in exact portion control (EPC), has become available.

It is thus an object of the present invention to provide a method in which a user is able to capture an 3D imaging of a meal and have an application with advanced image recognition capabilities, analyze, determine, and log the nutritional information, certain ingredients, allergenic ingredients, portion weights or size, as well as the percent DV of the nutrient intake that is based on a given caloric diet, as well having the ability to produce the 3D scale models of the meal or any individual component thereof for EPC.

### BRIEF DESCRIPTION OF THE DRAWINGS

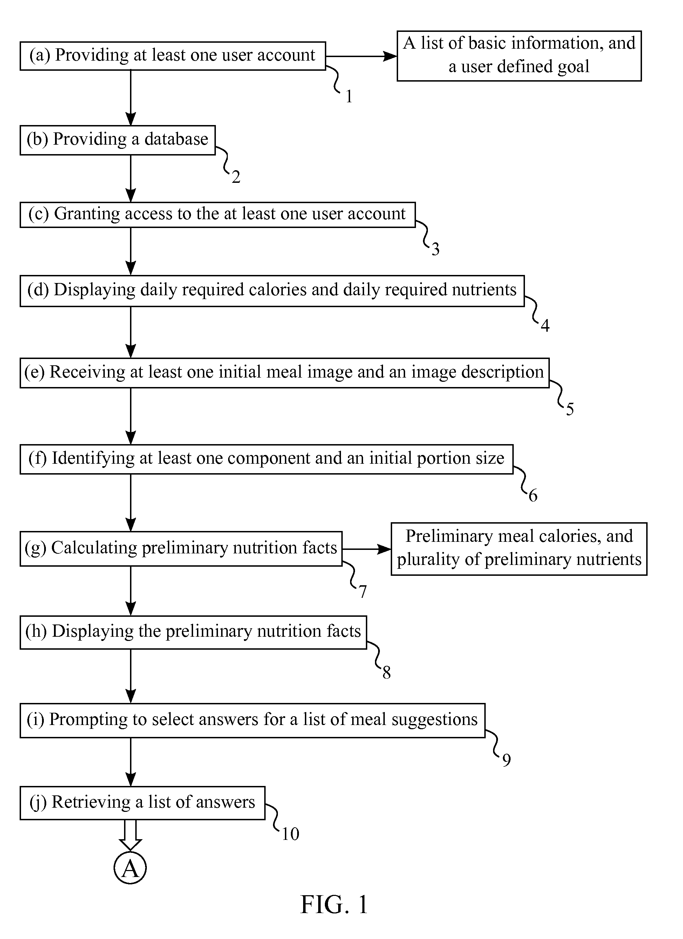

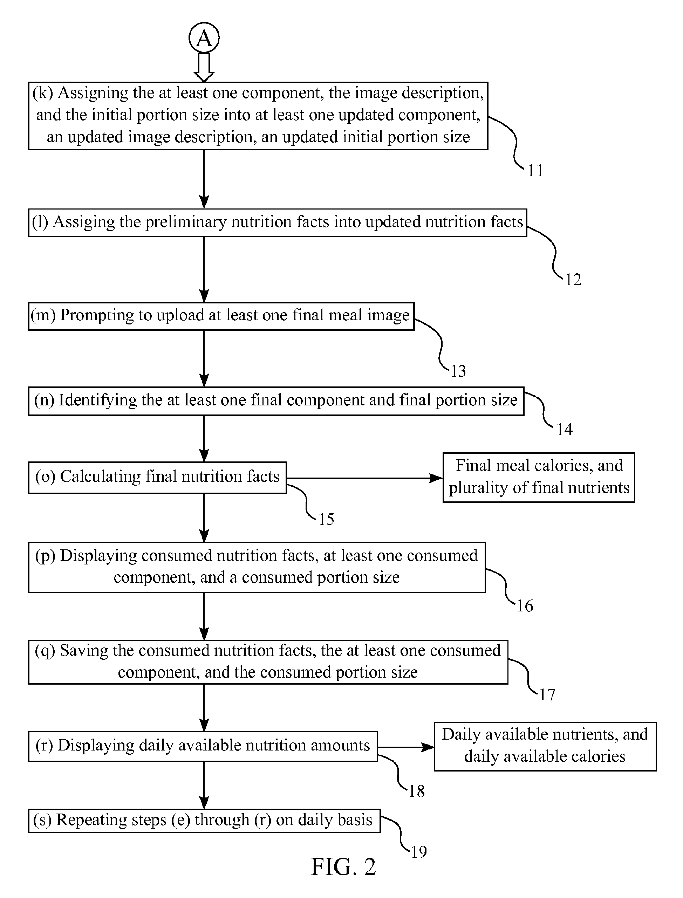

FIG. **1** is a flow chart illustrating an overall process of the present invention, wherein the letter A indicates that overall process of the FIG. **1** leads into FIG. **2****.**
FIG. **2** is a flow chart illustrating the overall process of the present invention, wherein the letter A indicates that the FIG. **2** is a continuation of the overall process in FIG. **1****.**

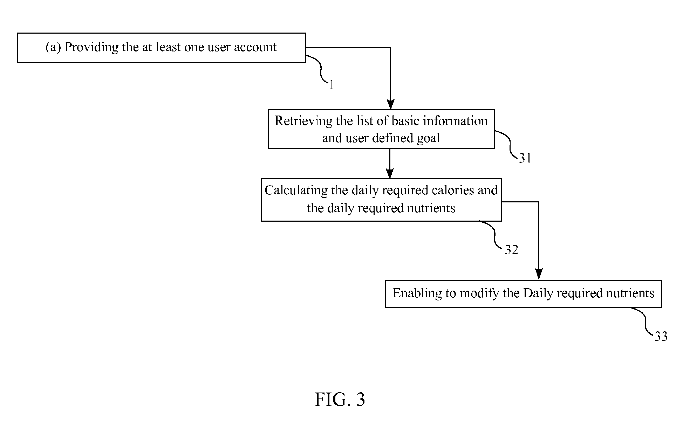

FIG. **3** is a flow chart illustrating the at least one user account creation within the overall process of the present invention.

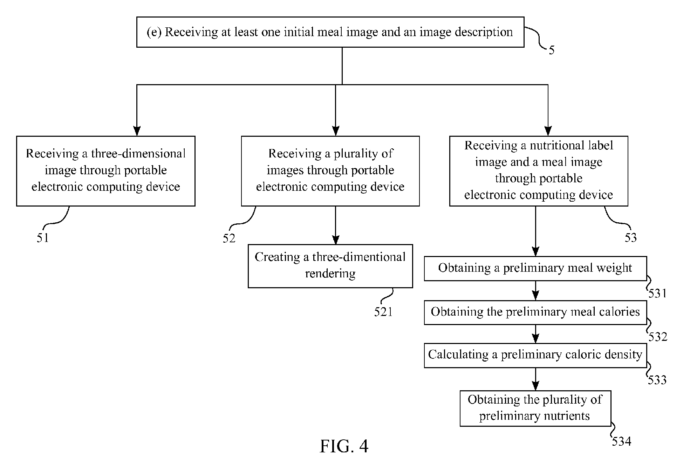

FIG. **4** is a flow chart illustrating different method of retrieving the at least one initial meal image within the overall process of the present invention.

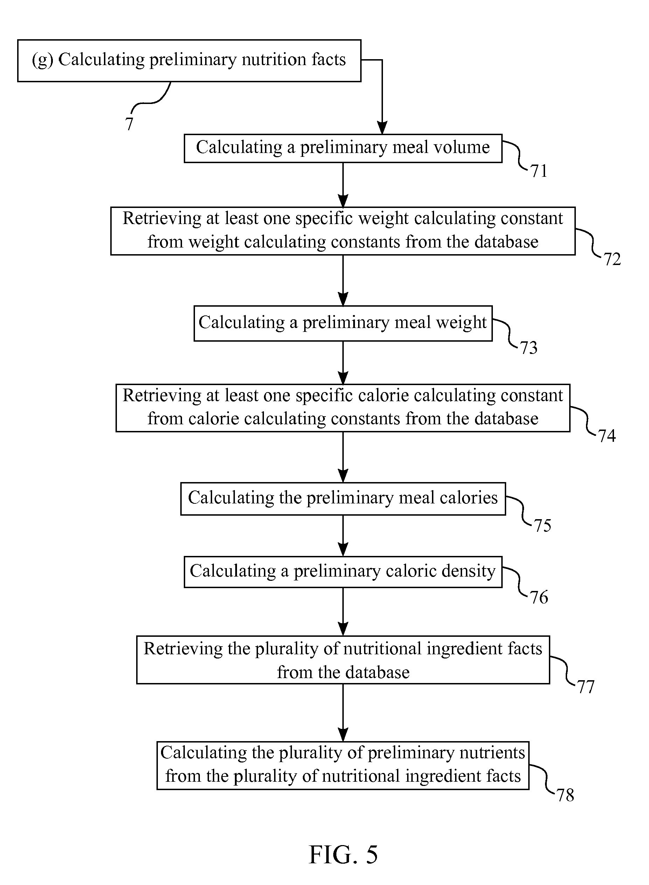

FIG. **5** is a flow chart illustrating the calculation of the preliminary nutrition facts within the overall process of the present invention.

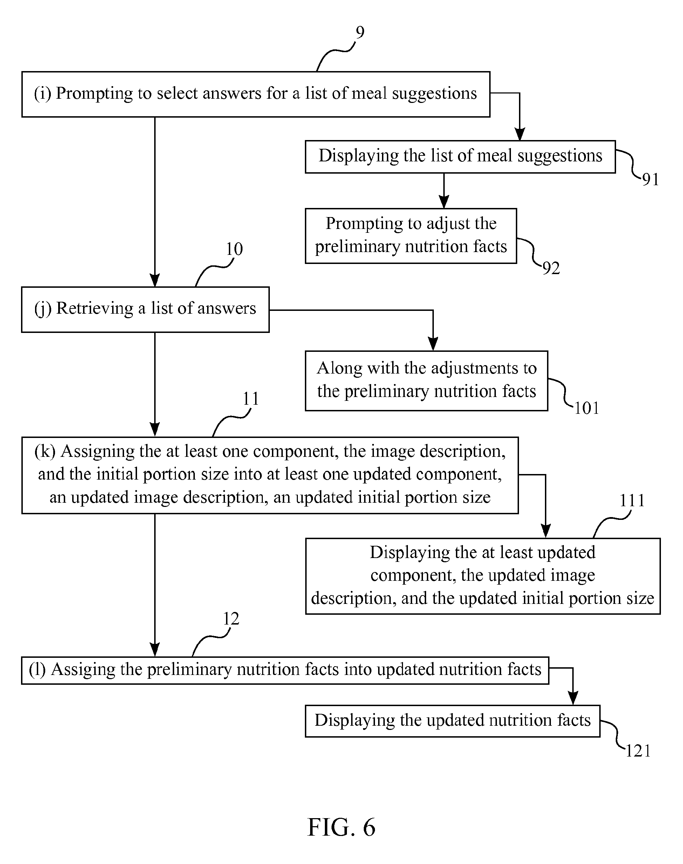

FIG. **6** is a flow chart illustrating the list of meal suggestions and the list of answers within the overall process of the present invention.

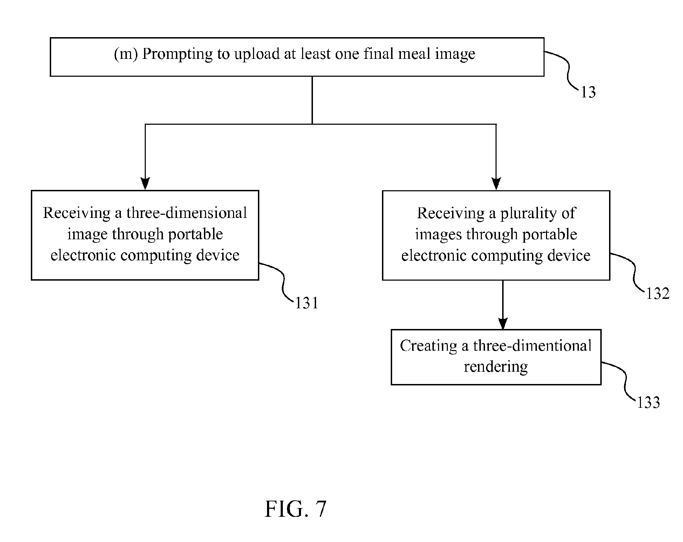

FIG. **7** is a flow chart illustrating different method of retrieving the at least one final meal image within the overall process of the present invention.

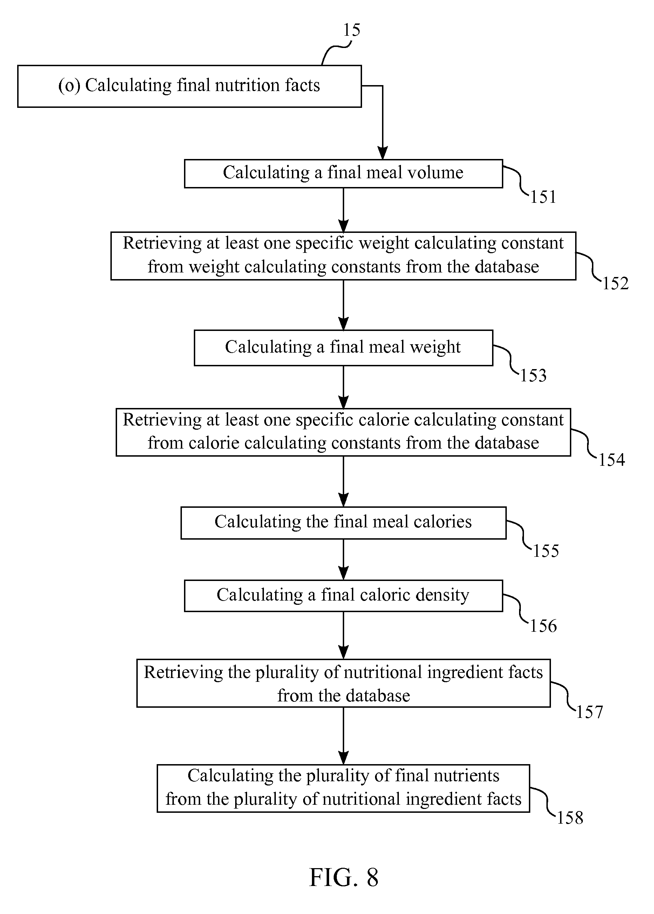

FIG. **8** is a flow chart illustrating the calculation of the final nutrition facts within the overall process of the present invention.

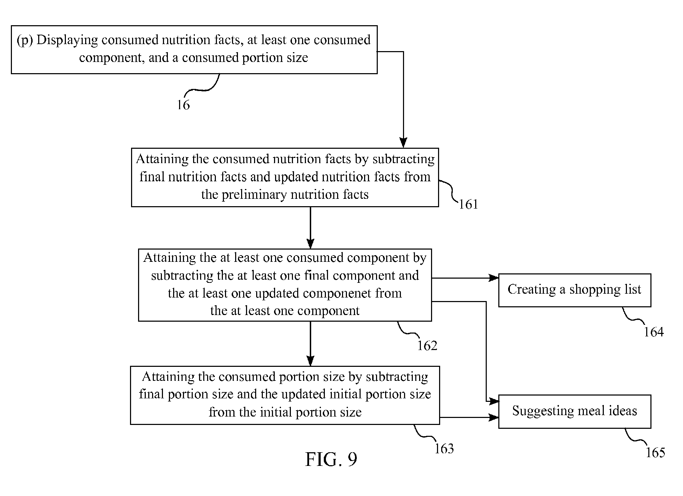

FIG. **9** is a flow chart illustrating the consumed nutrition facts, at least one component, and portion size within the overall process of the present invention.

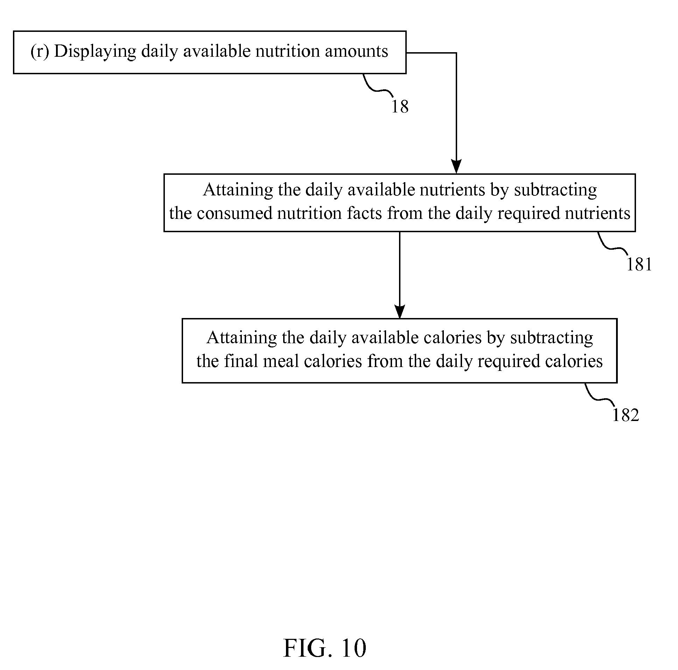

FIG. **10** is a flow chart illustrating the daily available nutrition amounts within the overall process of the present invention.
FIG. **11** is a flow chart illustrating the communicable connection of the database and the portable electronic computing devices.

### DETAIL DESCRIPTIONS OF THE INVENTION

All illustrations of the drawings are for the purpose of describing selected versions of the present invention and are not intended to limit the scope of the present invention.

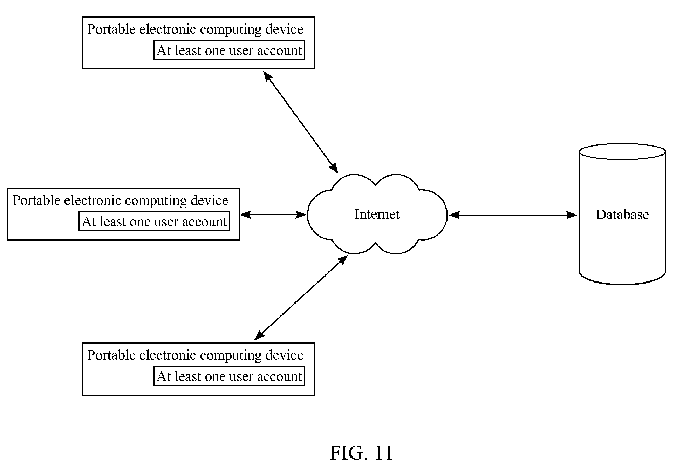

The present invention provides a fixed and or mobile application that is accessed through at least one user account. In reference to FIG. **11**, the at least one user account is accessed through a portable electronic computing device, where the portable electronic computing device is communicably coupled with a database. Even though the present invention is described within the at least one user account, a plurality of user accounts can individually and communicably coupled with the database through a plurality of electronic computing devices. The portable electronic computing device includes, but not limited to, smartphones, tablet computers, laptops and Laser scanners.

When the at least one user account is created by a user, the user needs to input a list of basic information and a user defined goal (step **1**). The list of basic information can include, but not limited to, name of the user, gender of the user, age of the user, height of the user, current weight of the user, desired weight of the user, allergenic ingredients, a user name, and a password. The user also needs to select the user defined goal from a list of pre-defined goals. More specifically, during the process of creating the at least one user account, the list of pre-defined goals is prompted to the user so that the user can select a preferred goal which considers as the user define goal within the present invention. For example, if the list of pre-defined goals includes three different goals, such as loss five pounds within two weeks, loss five pounds within three weeks, and maintain the current weight, the user selects maintain the current weight as the preferred goal. Then the maintaining the current weight becomes the user defined goal. When the present invention retrieves the list of basic information and the user defined goal (step **31**), the present invention calculates daily required calories and daily required nutrients for the at least one user account according to the list of basic information and the user defined goal (step **32**). Then the daily required calories and the daily required nutrients are displayed to the user through the portable electronic computing device (step **4**). The present invention also enables the user to modify the daily required nutrients through the portable electronic computing device (step **33**). For example, a diabetic patient normally wants to consume less amount of sugar compare to a healthy person. In such situation, the diabetic patient can modify the sugar level of the daily required nutrients. Once the at least one user account is created by the user, the at least one user account is stored in the database. Then the at least one user account can be accessed through the portable electronic computing device through the user name and the password (step **3**).

The database (step **2**) comprises weight calculating constants, calorie calculating constants, a plurality of nutritional ingredient facts, nutritional facts on pre-package food items, and nutritional facts on restaurant menu items. The nutritional facts on restaurant menu items in database include every available menu items from the restaurant industry, where the each of the menu items is organized by the respective restaurant name. The nutritional facts on pre-package food items in database include every available pre-package food items from the retail industry, where the each of the pre-package food items is organized by the respective brand name. The plurality of nutritional ingredient facts in the database provides information about all of the different nutritional ingredients, such as fat, saturated fat, trans fat, cholesterol, sodium, carbohydrates, dietary fiber, sugars, protein, vitamin A, vitamin C, calcium, and iron, so that the correct amount of nutritional ingredient can be calculated within the present invention. The calorie calculating constants, which provide the unit volume of the meal with relative to one calorie of the meal, are also stored within the database. For example, 1 calorie of boneless cooked ham has 0.04 cubic inches of meal volume. The weight calculating constants, which provide the unit volume of the meal with relative to one unit weight of the meal, are also stored within the database. For example, 1 ounce of chicken breast, which is the one unit weight of the meal, has 1.66 cubic inches of chicken breast, which is the unit volume of the meal.

When the user intends to consume a meal, which can be breakfast, lunch, dinner, snack, and intermediate meals, the user needs to take at least one initial meal image with the portable electronic computing device. The present invention then receives the at least one initial meal image and an image description through the portable electronic computing device (step **5**). The at least one initial meal image enables the present invention to identify at least one component and an initial portion size of the meal. In the present invention, the at least one initial meal image can be a three-dimensional (3D) image, a plurality of images, a (3D) Laser scan, a nutritional label image and a meal image, and a bar code image. The embedded technologies of the present invention, such as optical character recognition technology, the logical form recognition technology, and the advance image recognition technology, are able to clearly identify the at least one initial image within the present invention. The type of captured at least one initial meal image would depend on the capabilities of the fixed, and or portable, electronic computing device. For example, the portable electronic computing device that uses 3D image capturing would capture the 3D image, and the portable electronic computing device that uses standard image capturing would require capturing the plurality of images. If the present invention detects any of the allergenic or genetically modified ingredients as the at least one component, the present invention provides an alert to the at least one user account regarding the allergenic or genetically modified ingredients informing the user about the potential food allergy problems.

The present invention can receive the 3D image as the at least one initial meal image through the portable electronic computing device, where the 3D image allows the present invention to identify the at least one component and the initial portion size (step 51). Then the present invention identifies the at least one component and the initial portion size through the 3D image (step **6**). For example, if the meal is a plate of rice with chicken, the present invention identifies the rice and the chicken as the at least one component, and identifies unit dimensions on the initial portion size of the rice and the chicken. The unit dimensions can be length, height, width, or any other related dimensions which are required by the present invention to calculate the unit volume.

The present invention can receive the plurality of images as the at least one initial meal image through the portable electronic computing device, where the plurality of images allows the present invention to identify the at least one component and the initial portion size (step **52**). The plurality of images includes, but not limited to, top, front, rear, left, and right elevation view. Once the present invention receives the plurality of images, the present invention creates a three-dimensional rendering from the plurality of images (step **521**). Similar to the 3D image, the present invention is then able to identify the at least one component of the meal and the unit dimensions on the initial portion size (step **6**). If the user is dining out in a restaurant, the user can still upload the plurality of images, where the present invention enables the user to select a correct menu item from a specific restaurant through a list of restaurants. Once the correct menu item and the specific restaurant are selected and retrieved by the present invention, the present invention searches through the nutritional facts on restaurant menu items so that nutritional facts on correct menu item can be retrieved from the database, where the present invention is able to determine the at least one components and the initial portion size.

The present invention can retrieve the nutritional label image and the meal image as the at least one initial meal image through the portable electronic computing device, where the nutritional label image and the meal image allow the present invention to identify the at least one component and the initial portion size (step **53**). The initial portion size is easily identified through the nutritional label image since the initial portion size is stated. The at least one component of the meal is identified through the meal image and the nutritional label (step **6**).

The present invention can retrieve the bar code image as the at least one initial meal image through the portable electronic computing device, where the bar code image allows the present invention to identify the at least one component and the initial portion size. When the bar code image is received to the present invention through the portable electronic computing device, the present invention searches through the nutritional facts on pre-package food items so that nutritional facts on correct pre-package can be retrieved from the database, where the present invention is also able to determine the at least one components of the meal and the initial portion size.

The image description enables the present invention to clearly identify prepared method of the at least one component. For example, if the user is having baked chicken for dinner, the user can upload the at least one initial meal image with the image description, where the image description states that the chicken is baked. Since different meal preparations create different nutrition facts, the image description provide valuable information to the present invention so that correct identification can take place for the at least one component. The image description can be uploaded into the present invention as text commands or voice commands. The present invention may also prompt additional questions to clearly identify the prepared method and the at least one component if the provided information is not sufficient.

Once the present invention identifies the at least one component and the initial portion size, the present invention then calculates preliminary nutrition facts with reference to the at least one component, the image description, and the initial portion size, where the preliminary nutrition facts comprise preliminary meal calories and a plurality of preliminary nutrients (step **7**).

When the present invention retrieves the 3D image or the plurality of images as the at least one initial meal image, the present invention calculates a preliminary meal volume of the at least one component according to the initial portion size, using the unit dimensions (step **71**). The preliminary meal volume is calculated by either using the length, the width, and the height of the portion size or using the area and the height of the portion size. Depending on the shape of the initial portion size, the present invention can use any other type of mathematical algorithm to calculate the preliminary meal volume. Then the present invention retrieves at least one specific weight calculating constant from the weight calculating constants, where the at least one specific weight calculating constant correlates with the at least one component (step **72**). For example, if the meal comprises potatoes and green beans as the at least one component, a potato weight calculating constant and a green beans weight calculating constant as the at least one specific weight calculating constant are retrieved from the database. Then a preliminary meal weight is calculated from the preliminary meal volume and the at least one specific weight calculating constant (step **73**). More specifically, the preliminary meal volume is divided by the at least one specific weight calculating constant so that the preliminary meal weight can be obtained. Following is an example that illustrates the calculation process of the preliminary meal weight within the present invention.

### Example 1:

The unit dimensions of a chicken breast: 5 in x 2 in x 1 in

The preliminary meal volume: 5 in x 2 in x 1 in = 10 in³

The at least one specific weight calculating constant for chicken breast: 1.66 in³ per oz

The preliminary meal weight: 10 in³ / 1.66 in³ per oz = 6 oz

Then the preliminary meal calories are calculated from the preliminary meal volume and at least one specific calorie calculating constant from the calorie calculating constants. Similar to the at least one specific weight calculating constant, the at least one specific calorie calculating constant is also correlated with the at least one component. More specifically, the present invention retrieves the at least one specific calorie calculating constant from the database (step **74**), and then the preliminary meal volume is divided by the at least one specific calorie calculating constant to obtain the preliminary meal calories (step **75**). Following is an example that illustrates the calculation process of the preliminary meal calories within the present invention.

### Example 2:

The unit dimensions of a chicken breast: 5 in x 2 in x 1 in

The preliminary meal volume: 5 in x 2 in x 1 in = 10 in³

The at least one specific calorie calculating constant for chicken breast: 0.07 in³ per calorie

The preliminary meal calories: 10 in³ / 0.07 in³ per calorie = 143 calories

The present invention then calculates a preliminary caloric density, where the preliminary meal calories are divided by the preliminary meal weight to attain the preliminary caloric density (step **76**). The preliminary caloric density is a measurement of the average calories per unit weight of the at least one component. The preliminary caloric density provides platform for the user about how much food can be consumed while staying within a specific amount of calories. For example, if the preliminary caloric density is low within a food item, the user can consume bigger portion of that food item and stay within the specific amount of calories. If the preliminary caloric density is high within a food item, the user has to consume smaller portion of that food item in order to stay within the specific amount of calories.

The present invention then retrieves the plurality of nutritional ingredient facts from the database according to the at least one component (step **77**). For example, if the at least one component is a piece of gum, the present invention retrieves carbohydrate and sugar from the database as the plurality of nutritional ingredient facts since the piece of gum only contains those nutritional facts. Then the present invention calculates the plurality of preliminary nutrients for the at least one component according to the initial portion size (step **78**). For example, if the one piece of gum comprises 2 grams of sugar and the identified portion size is 4 pieces of gum, the calculated plurality of preliminary nutrients for the 4 pieces of gum are 8 grams of sugar.

When the present invention retrieves the nutritional label image and a meal image, the present invention obtains the preliminary meal weight (step **531**) and the preliminary meal calories (step **532**) from the nutritional label image. Then the present invention is able to calculate the preliminary caloric density by dividing the preliminary meal calories from the preliminary meal weight (step **533**). The present invention also obtains the plurality of preliminary nutrients from the nutritional label image (step **534**).

When the present invention retrieves the bar code image or the correct menu item from the specific restaurant, the present invention is able to obtain the preliminary meal volume, the preliminary meal calories, the preliminary caloric density, and the plurality of preliminary nutrients from the database since all of these information are respectively stored within the nutritional facts on pre-package food items and the nutritional facts on restaurant menu items.

Once the preliminary nutrition facts are calculated, the preliminary nutrition facts are displayed to the user through the portable electronic computing device with a perspective image of the meal (step **8**). Simultaneously, the present invention prompts a list of meal suggestions to the user through the portable electronic computing device (step **9**). When the list of meal suggestions is displayed through the portable electronic computing device, the user is able to see the list of meal suggestions (step **91**). The list of meal suggestions prompts the user to adjust the preliminary nutrition facts according to the preliminary meal calories, the plurality of preliminary nutrients, the initial portion size, and the preliminary meal weight (step **92**). The list of meal suggestions may propose the user take out at least one component or a portion of the at least one component so that the preliminary nutrition facts can be altered if the user feels necessary. For example, if the meal comprises rice and chicken, the list of meal suggestions can recommend the user to take off half of the rice. The list of suggestions can include a plurality of advices, where the plurality of advices provides different options to the user so that the preliminary nutrition facts can be lowered while aiding the user to achieve the user defined goal. Once the user provides answers to the list of meal suggestions, a list of answers for the list of meal suggestions is retrieves to the present invention (step **10**), where the present invention provides adjustments to the preliminary nutrition facts according to the list of answers (step **101**).

Then the present invention assigns the at least one component, the image description, and the initial portion size into at least one updated component, an updated image description, an updated initial portion size according to the list of answers (step **11**). For example, if the user decides to take off half of the rice, the at least one updated component, the updated image description, and the updated initial portion size differ from the at least one component, the image description, and the initial portion size. Then the preliminary nutrition facts also get assign into updated nutrition facts according to the list of answers since the at least one component and the portion size is changed (step **12**). The at least one updated component, the updated image description, the updated initial portion size, and the updated nutrition facts are then displayed through the portable electronic computing device (step **111**) (step **121**).

The present invention prompts the user to upload at least one final meal image through the portable electronic computing device, where the at least one final meal image is uploaded after the meal is consumed by the user (step **13**). The at least one final meal image is put in place within the present invention so that if the user decides to leave out any leftover food items from the meal, the present invention can take those leftover food items into consideration, providing accurate calculations. The at least one final meal image enables the present invention to identify at least one final component and a final portion size of the at least one final meal image. Similar to the at least one initial meal image, the at least one final meal image within the present invention can be the 3D image (step **131**) and the plurality of images (step **132**), where the plurality of images creates a three-dimensional rendering (step **133**). When the at least one final meal image retrieves to the present invention, the present invention identifies the at least one final component and the final portion size (step **14**). Then the present invention calculates final nutrition facts for the at least one final component and the final portion size, wherein the final nutrition facts comprise final meal calories and a plurality of final nutrients (step **15**). Similar to the at least one initial meal image, the at least one final meal image calculates a final meal volume of the at least one final component (step **151**). Then a final meal weight is calculated by dividing the final meal volume by the at least one specific weight calculating constant (step **153**), where the at least one specific weight calculating constant retrieves from the weight calculating constants and correlates with the at least one final component (step **152**). Then the at least one specific calorie calculating constant, which correlates with the at least one final component, is retrieved from the calorie calculating constants (step **154**). The final meal calories are calculated from the final meal volume and the at least one specific calorie calculating constant (step **155**). More specifically, the final meal volume is divided by the at least one specific calorie calculating constant to obtain the final meal calories. Then a final caloric density is calculated from the final meal weight and the final meal calories, wherein the final meal calories are divided by the final meal weight to obtain the final caloric density (step **156**). Then the present invention retrieves the plurality of nutritional ingredient facts from the database according to the at least one final component (step **157**), and calculates the plurality of final nutrients according to the final portion size (step **158**).

Then the present invention displays consumed nutrition facts, at least one consumed components, and a consumed portion size to the user through the portable electronic computing device (step **16**). The consumed nutrition facts are attained by subtracting the final nutrition facts and the updated nutrition facts from the plurality of preliminary nutrition facts (step **161**). The at least one consumed component is attained by subtracting the at least one final component and the at least one updated component from the at least one component (step **162**). The consumed portion size is attained by subtracting the updated initial portion size and the final portion size from the initial portion size (step **163**). Then the consumed nutrition facts, the at least one consumed component, and the consumed portion size are stored within the portable electronic computing device (step **17**). The stored information can also be downloaded into the user's home computer from the portable electronic computing device, where the home computer comprises complementary software and programs so that the information can be displayed to the user. The consumed nutrition facts, the at least one consumed component, and the consumed portion size can be displayed through different type graphs and chart so that the user can easily identify the information. The consumed nutrition facts, the at least one consumed component, and the consumed portion are also organized into predetermine time periods, where the predetermine time periods can be daily, weekly, monthly, yearly. The predetermine time periods allows the user keep track of the consumed nutrition facts, the at least one consumed component, and the consumed portion so that the progression toward the user defined goal can be achieved smoothly.

Then daily available nutrition amounts are displayed to the at least one user account through the portable electronic computing device, where the daily available nutrition amounts comprises daily available nutrients and daily available calories (step **18**). The present invention attains the daily available nutrients by subtracting the consumed nutrient facts from the daily required nutrients (step **181**) and attains the daily available calories by subtracting the final meal calories from the daily required calories (step **182**). Every time the user activates the at least one user account, the daily available nutrients and the daily available calories are displayed to the user. The whole process of the present invention from uploading at least one initial meal image to displaying of the daily available nutrients and the daily available calories needs to be processed on a daily basis every time the user decides to consume a meal so that accurate information can be stored within the present invention (step **19**).

Since most of the people repeatedly eat the same kind of food items, the present invention is able recognize the food items and log the food items into a shopping list after a pre-determine time period, according to the at least one consumed component and the user defined goals (step **164**). The shopping list provides a reference guide to the user so that grocery shopping can be relatively easy for the user. After the pre-determine time period, the present invention is also able to suggest meal ideas for the at least one user account (step **165**). The meal ideas are suggested according to the at least one consumed component and the consumed portion size.

If the user decides to do any kind of physical exercise, the present invention is also able to calculate burnt calories which are achieved through the physical exercise. The burnt calories are calculated according to user input information, which is inputted through the portable electronic computing device and explains the nature of the physical exercise, and the burnt calories are added to the daily available calories in order to balance the daily required nutrients.

The unit dimensions of the present invention can be used to produce 3D models and scales of meals through digital direct model printing so that the models and scales can be used for institutional proposes within culinary schools or any other meal preparation establishments, such as nursing homes, hospitals, professional & collegiate sport teams, motor sports teams, and household to aid in exact portion control. Since the unit dimensions provide an exact replica of the meal, the models and scales created by the digital direct model printing provide accurate information, eliminating food wastage. The unit dimension can also be used by food preparing industry to determine correct packaging sizes and food costing, and by table ware industry to determine correct table ware and glassware selections. Since the present invention identifies the at least one component and the initial portion size, the present invention can be used within the restaurant industry to manage inventory, manage food costing, produce on site nutrition fact labels. These different aspects of the present invention provide wide variety of functionality within the present invention.

Although the invention has been explained in relation to its preferred embodiment, it is to be understood that many other possible modifications and variations can be made without departing from the spirit and scope of the invention as hereinafter claimed.

## Claims

1. A method of creating a diet plan with three-dimensional imaging by executing computer-executable instructions stored on a non-transitory computer-readable medium, the method comprises the steps of:
(a) providing at least one user account (1), wherein the at least one user account comprises a list of basic information and a user defined goal;
(b) providing a database (2), wherein the at least one user account is stored in the database, and the database comprises weight calculating constants, calorie calculating constants, and a plurality of nutritional ingredient facts;
(c) granting access to the at least one user account (3) through a portable electronic computing device, wherein the portable electronic computing device is communicably coupled with the database;
(d) displaying daily required calories and daily required nutrients (4) through the portable electronic computing device;
(e) receiving at least one initial meal image and an image description (5) through the portable electronic computing device;
(f) identifying at least one component and an initial portion size (6) of the at least one initial meal image;
(g) calculating preliminary nutrition facts (7) for the at least one component, the image description, and the initial portion size, wherein the preliminary nutrition facts comprise preliminary meal calories and a plurality of preliminary nutrients;
(h) displaying the preliminary nutrition facts (8) through the portable electronic computing device;
(i) simultaneously, prompting to select answer for a list of meal suggestions (9) through the portable electronic computing device;
(j) retrieving a list of answers (10) for the list of meal suggestions;
(k) assigning the at least one component, the image description, and the initial portion size into at least one updated component, an updated image description, an updated initial portion size (11) according to the list of answers;
(l) assigning the preliminary nutrition facts into updated nutrition facts (12) according to the list of answers;
(m)prompting to upload at least one final meal image (13) through the portable electronic computing device;
(n) identifying the at least one final component and a final portion size (14) of the at least one final meal image;
(o) calculating final nutrition facts (15) for the at least one final component and the final portion size, wherein the final nutrition facts comprises final meal calories and a plurality of final nutrients;
(p) displaying consumed nutrition facts, at least one consumed component, and a consumed portion size (16);
(q) saving the consumed nutrition facts, the at least one consumed component, and the consumed portion size (17) in the portable electronic computing device;
(r) displaying daily available nutrition amounts (18) through the portable electronic computing device, wherein the daily available nutrition amounts comprises daily available nutrients and daily available calories; and
(s) repeating steps (e) through (r) on a daily basis (19).

2. A method of creating a diet plan as claimed in claim 1, comprises the steps of:
retrieving the list of basic information and the user defined goal (31); and
calculating the daily required calories and the daily required nutrients (32) for the at least one user account from the list of basic information and the user defined goal.

3. A method of creating a diet plan as claimed in claim 2, comprises the step of:
enabling to modify the daily required nutrients (33).

4. A method of creating a diet plan as claimed in claim 1, comprises the steps of:
receiving a three-dimensional image as the at least one initial meal image through the portable electronic computing device (51); and
identifying the at least one component and the initial portion size of the three-dimensional image (6).

5. A method of creating a diet plan as claimed in claim 1, comprises the steps of:
receiving a plurality of images as the at least one initial meal image through the portable electronic computing device (52);
creating a three-dimensional rendering (521) from the plurality of images; and
identifying the at least one component and the initial portion size of the three-dimensional rendering (6).

6. A method of creating a diet plan as claimed in claim 1, comprises the steps of:
receiving a nutritional label image and a meal image as the at least one initial meal image through the portable electronic computing device (53); and
identifying the at least one component and the initial portion size of the meal image (6).

7. A method of creating a diet plan as claimed in claim 1, comprises the steps of:
calculating a preliminary meal volume (71) of the at least one component according to the initial portion size, wherein the preliminary meal volume is obtained by multiplication of an area and a height of the at least one component;
retrieving at least one specific weight calculating constant form the weight calculating constants (72), wherein the at least one specific weight calculating constant correlates with the at least one component;
calculating a preliminary meal weight (73), wherein the preliminary meal volume is divided by the at least one specific weight calculating constant to obtain the preliminary meal weight;
retrieving at least one specific calorie calculating constant from the calorie calculating constants (74), wherein the at least one specific calorie calculating constant correlates with the at least one component;
calculating the preliminary meal calories (75), wherein the preliminary meal volume is divided by the at least one specific calorie calculating constant to obtain the preliminary meal calories;
calculating a preliminary caloric density (76) from the preliminary meal weight and the preliminary meal calories, wherein the preliminary meal calories are divided by the preliminary meal weight to obtain the preliminary caloric density;
retrieving the plurality of nutritional ingredient facts from the database (77) according to the at least one component; and
calculating the plurality of preliminary nutrients from the plurality of nutritional ingredient facts (78) according to the initial portion size.

8. A method of creating a diet plan as claimed in claim 6, comprises the steps of:
obtaining a preliminary meal weight (531) from the nutritional label image;
obtaining the preliminary meal calories (532) from the nutritional label image;
calculating a preliminary caloric density (533) from the preliminary meal weight and the preliminary meal calories, wherein the preliminary meal calories are divided by the preliminary meal weight to obtain the preliminary caloric density; and
obtaining the plurality of preliminary nutrients (534) from the nutritional label image.

9. A method of creating a diet plan as claimed in claim 1, comprises the steps of:
displaying the list of meal suggestions (91) through the portable electronic computing device;
prompting to adjust the preliminary nutrition facts (92) according to the preliminary meal calories, the plurality of preliminary nutrients, the initial portion size, and a preliminary meal weight;
retrieving the list of answers along with the adjustments to the preliminary nutrition facts (101);
displaying the at least one updated component, the updated image description, the updated initial portion size (111) through the portable electronic computing device; and
displaying the updated nutrition facts (121) through the portable electronic computing device.

10. A method of creating a diet plan as claimed in claim 1, comprises the steps of:
retrieving a three-dimensional image (131) as the at least one final meal image through the portable electronic computing device; and
identifying the at least one final component and the final portion size of the three-dimensional image (14).

11. A method of creating a diet plan as claimed in claim 1, comprises the steps of:
retrieving a plurality of images (132) as the at least one final meal image through the portable electronic computing device;
creating a three-dimensional rendering (133) from the plurality of images; and
identifying the at least one final component and the final portion size of the three-dimensional rendering (14).

12. A method of creating a diet plan as claimed in claim 1, comprises the steps of:
calculating a final meal volume (151) of the at least one final component according to the final portion size, wherein the final meal volume is obtained by multiplication of an area and a height of the at least one final component;
retrieving at least one specific weight calculating constant form the weight calculating constants (152), wherein the at least one specific weight calculating constant correlates with the at least one final component;
calculating a final meal weight (153), wherein the final meal volume is divided by the at least one specific weight calculating constant to obtain the final meal weight;
retrieving at least one specific calorie calculating constant from the calorie calculating constants (154), wherein the at least one specific calorie calculating constant correlates with the at least one final component;
calculating the final meal calories (155), wherein the final meal volume is divided by the at least one specific calorie calculating constant to obtain the final meal calories;
calculating a final caloric density (156) from the final meal weight and the final meal calories, wherein the final meal calories are divided by the final meal weight to obtain the final caloric density;
retrieving the plurality of nutritional ingredient facts from the database (157) according to the at least one final component; and
calculating the plurality of final nutrients from the plurality of nutritional ingredient facts (158) according to the final portion size.

13. A method of creating a diet plan as claimed in claim 1, comprises the steps of:
attaining the consumed nutrition facts by subtracting the final nutrition facts and the updated nutrition facts from the preliminary nutrition facts (161);
attaining the at least one consumed component by subtracting the at least one final component and the at least one updated component from the at least one component (162); and
attaining the consumed portion size by subtracting the final portion size and the initial portion size from the initial portion size (163).

14. A method of creating a diet plan as claimed in claim 13, comprises the steps of:
creating a shopping list for the at least one user account within the completion of a pre-determine time period according to the at least one consumed component (164); and
suggesting meal ideas for the at least one user account within the completion of the pre-determine time period according to the at least one consumed component and the consumed portion size (165).

15. A method of creating a diet plan as claimed in claim 1, comprises the steps of:
attaining the daily available nutrients by subtracting the consumed nutrition facts from the daily required nutrients (181); and
attaining the daily available calories by subtracting the final meal calories from the daily required calories (182).
